# EUROPÄISCHE PATENTSCHRIFT

(11) **EP 1 831 240 B1**
(45) Veröffentlichungstag und Bekanntmachung des Hinweises auf die Patenterteilung: **15.07.2009**
(21) Anmeldenummer: 05850392.1
(22) Anmeldetag: 30.12.2005
(51) Int. Cl.: C07J 53/00, A61K 31/585, A61P 5/34

(54) **18-METHYL-19-NOR-17-PREGN-4-EN-21,17-CARBOLACTONE, SOWIE DIESE ENTHALTENDE PHARMAZEUTISCHE PRÄPARATE**
18-METHYL-19-NOR-17-PREGN-4-EN-21,17-CARBOLACTONES AND PHARMACEUTICAL PREPARATIONS COMPRISING THE SAME
18-METHYL-19-NOR-17-PREGN-4-EN-21,17-CARBOLACTONES ET PREPARATIONS PHARMACEUTIQUES CONTENANT CES DERNIERS

(30) Priorität: 30.12.2004 DE 102004063864
(43) Veröffentlichungstag der Anmeldung: 12.09.2007
(73) Patentinhaber: Schering Aktiengesellschaft, 13353 Berlin (DE)
(72) Erfinder: BOHLMANN, Rolf, 14055 Berlin (DE); BITTLER, Dieter, 13503 Berlin (DE); KUENZER, Hermann, 13347 Berlin (DE); ESPERLING, Peter, 12107 Berlin (DE); MUHN, Hans-Peter, 13465 Berlin (DE); FRITZEMEIER, Karl-Heinrich, 13503 Berlin (DE); FUHRMANN, Ulrike, 10587 Berlin (DE); PRELLE, Katja, 13465 Berlin (DE); BORDEN (FRÜHER KURZ), Steffen, 13355 Berlin (DE)
(86) Internationale Anmeldenummer: PCT/EP2005/014205
(87) Internationale Veröffentlichungsnummer: WO 2006/072467

(56) Entgegenhaltungen:
- DE-A1- 3 022 337
- DE-A1- 3 402 329
- NICKISCH, K. ET AL: "Aldosterone antagonists. 4. Synthesis and activities of steroidal 6,6-ethylene-15,16-methylene 17-spirolactones" JOURNAL OF MEDICINAL CHEMISTRY, Bd. 34, 1991, Seiten 2464-2468, XP002375413

## Beschreibung

Die vorliegende Erfindung betrifft 18-Methyl-19-nor-17-pregn-4-en-21,17-carbolactone der allgemeinen Formel I 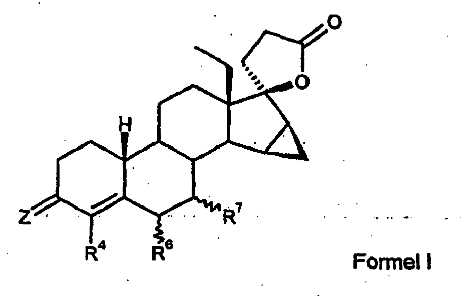 worin
Z ein Sauerstoffatom, zwei Wasserstoffatome, eine Gruppierung =NOR oder =NNHSO₂R, wobei R ein Wasserstoffatom oder eine gerad- oder verzweigtkettige Alkylgruppe mit 1 bis 4 bzw. 3 bis 4 Kohlenstoffatomen ist,
R⁴ ein Wasserstoffatom, ein Halogenatom, eine Methyl- oder eine Trifluormethylgruppe,
R⁶ und/oder R⁷ α- oder β-ständig sein kann und unabhängig voneinander eine gerad- oder verzweigtkettige Alkylgruppe mit 1 bis 4 bzw. 3 bis 4 Kohlenstoffatomen oder R⁶ ein Wasserstoffatom und R⁷ eine α- oder β-ständige, gerad- oder verzweigtkettige Alkylgruppe mit 1 bis 4 bzw. 3 bis 4 Kohlenstoffatomen oder
R⁶ und R⁷ gemeinsam eine α- oder β-ständige Methylengruppe oder eine zusätzliche Bindung
bedeuten.

Z steht vorzugsweise für ein Sauerstoffatom.

Im Falle, daß Z für eine Gruppierung =NOR oder =NNHSO₂R steht, ist R vorzugsweise ein Wasserstoffatom.
Für eine gerad- oder verzweigtkettige Alkylgruppe mit 1 bis 4 bzw. 3 bis 4 Kohlenstoffatomen kommt eine Methyl-, Ethyl-, n-Propyl- oder eine n-Butylgruppe bzw. eine iso-Propyl-, iso- oder tert.-Butylgruppe in Betracht.

R⁴ ist vorzugsweise ein Wasserstoffatom.
Als Halogenatom R⁴.kommt ein Fluor-, Chlor-, Brom- oder lodatom in Frage; Chlor ist unter diesen bevorzugt.

Im Falle, daß R⁶ und/oder R⁷ eine gerad- oder verzweigtkettige Alkylgruppe mit 1 bis 4 bzw. 3 bis 4 Kohlenstoffatomen ist, kommt hierfür eine Methyl-, Ethyl-, n-Propyl- oder eine n-Butylgruppe bzw. eine iso-Propyl-, iso- oder tert.-Butylgruppe in Betracht.
R⁶ und R⁷ stehen vorzugsweise für ein Wasserstoffatom und eine Methylgruppe oder gemeinsam für eine Methylengruppe oder eine Doppelbindung.

Nachstehend genannte Verbindungen sind erfindungsgemäß besonders bevorzugt:
18-Methyl-15β,16β-methylen-3-oxo-19-nor-17-pregna-4,6-dien-21,17-carbolacton
18-Methyl-6α,7α-15β,16β-dimethylen-3-oxo-19-nor-17-pregn-4-en-21,17-carbolacton
18-Methyl-6β,7β-15β,16β-dimethylen-3-oxo-19-nor-17-pregn-4-en-21,17-carbolacton
7α,18-Dimethyl-15β,16β-methylen-3-oxo-19-nor-17-pregn-4-en-21,17-carbolacton
7β,18-Dimethyl-15β,16β-methylen-3-oxo-19-nor-17-pregn-4-en-21,17-carbolacton
3-Hydroxylamino-18-methyl-6β,7β-15β,16β-dimethylen-19-nor-17-pregn-4-en-21,17-carbolacton
4-Chlor-18-methyl-6β,7β-15β,16β-dimethylen-3-oxo-19-nor-17-pregn-4-en-21,17-carbolacton
7α-Ethyl-18-methyl-15β,16β-methylen-3-oxo-19-nor-17-pregn-4-en-21,17-carbolacton
7β-Ethyl-18-methyl-15β,16β-methylen-3-oxo-19-nor-17-pregn-4-en-21,17-carbolacton
18-Methyl-15β,16β-methylen-3-oxo-7α-propyl-19-nor-17-pregn-4-en-21,17-carbolacton
18-Methyl-15β,16β-methylen-3-oxo-7β-propyl-19-nor-17-pregn-4-en-21,17-carbolacton

Drospirenon (6β,7β-15β,16β-Dimethylen-3-oxo-17-pregn-4-en-21,17β-carbolacton) ist ein neues Gestagen, welches beispielsweise in dem oralen Kontrazeptivum YASMIN^{®} und dem Präparat ANGELIQ^{®} zur Behandlung postmenopausaler Beschwerden (beide SCHERING AG) enthalten ist (DE 3022337). Aufgrund seiner vergleichsweise geringen Affinität zum Gestagenrezeptor und seiner vergleichsweise hohen Ovulationshemmdosis ist

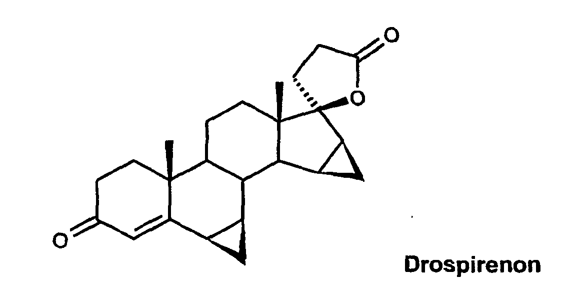

Drospirenon in YASMIN^{®} in der relativ hohen täglichen Dosis von 3 mg enthalten. Drospirenon zeichnet sich dadurch aus, daß es zusätzlich zur gestagenen Wirkung über aldosteronantagonistische (antimineralcorticoide) sowie antiandrogene Wirkung verfügt. Diese beiden Eigenschaften machen das Drospirenon in seinem pharmakologischen Profil dem natürlichen Gestagen Progesteron sehr ähnlich, welches aber anders als das Drospirenon nicht ausreichend oral bioverfügbar ist.

Aufgabe der vorliegenden Erfindung ist es deshalb, Verbindungen zur Verfügung zu stellen, die über eine stärkere Bindung an den Gestagenrezeptor verfügen als das Drospirenon und somit über höhere gestagene Potenz als das Drospirenon verfügen sollen. Dies soll sich letztlich in einer geringeren täglichen Dosierung manifestieren und zu einem geringeren Substanzbedarf an aktiver Verbindung führen.

Diese Aufgabe wird durch die Bereitstellung der hier beschriebenen 18-Methyl-19-nor-17-pregn-4-en-21,17-carbolactone der allgemeinen Formel I gelöst. Die Verbindungen der allgemeinen Formel I (und insbesondere die Verbindung 3b, siehe exp. Teil) sind als Konstitutionsisomere des Drospirenons anzusehen. Die neuen Verbindungen zeichnen sich im Gestagenrezeptor-Bindungstest unter Verwendung von Cytosol aus Kaninchenuterushomogenat und von 3H-Progesteron als Bezugssubstanz durch eine höhere Affinität zum Gestagenrezeptor als Drospirenon und durch vergleichbare Affinität zum Mineralocorticoidrezeptor aus Rattennierenhomogenat aus.
Die Bindung an den Gestagenrezeptor ist dabei überraschenderweise bis zu fünf mal so stark wie die des Drospirenons.

Die erfindungsgemäßen Verbindungen zeichnen sich überraschenderweise durch starke gestagene Wirksamkeit aus und sind im Schwangerschaftserhaltungs-Test an der Ratte nach subcutaner Applikation stark wirksam.

Durchführung des Schwangerschaftserhaltungstests an der Ratte:
In graviden Ratten induziert die Entfernung der Corpora lutea oder Kastration einen Abort. Durch die exogene Zufuhr von Progestinen (Gestagenen) in Kombination mit einer geeigneten Dosis eines Estrogens, gelingt die Aufrechterhaltung der Schwangerschaft. Der Schwangerschaftserhaltungstest an ovarektomierten Ratten dient zur Bestimmung der peripheren gestagenen Aktivität einer Verbindung.

Ratten werden während des Proestrus über Nacht angepaart. Die Anpaarung wird am Morgen des darauf folgenden Tages durch die Begutachtung eines Vaginalabstriches kontrolliert. Die Anwesenheit von Spermien wird dabei als Tag 1 einer beginnenden Schwangerschaft bewertet. Am Tag 8 der Schwangerschaft werden die Tiere unter Etheranesthesie ovarektomiert. Die Behandlung mit Testverbindung und exogenem Estrogen (Estron, 5 µg/kg/Tag) wird von Tag 8 bis Tag 15 oder Tag 21 der Schwangerschaft einmal täglich subkutan ausgeführt. Die erste Anwendung am Tag 8 wird 2 Stunden vor der Kastration ausgeführt. Intakte Kontrolltiere erhalten ausschließlich Vehikel.

### Auswertung:

Am Ende des Versuches (Tag 15 oder Tag 21) werden die Tiere unter CO₂-Atmosphäre getötet und es werden lebende Föten (Föten mit schlagendem Herz) und Implantationsstellen (frühe Resorptionen und tote Föten einschließlich Autolyse und atrophische Plazenten) in beiden uterinen Hömem gezählt. Am Tag 22 können Föten außerdem auf Missbildungen untersucht werden. In Uteri ohne Föten oder Implantationsstellen wird die Anzahl von Nidationsstellen durch Anfärben mit 10%iger Ammoniumsulfid-Lösung ermittelt. Die Schwangerschaftserhaltungsrate wird als Quotient aus der Anzahl der lebenden Föten und der gesamten Anzahl von Nidationsstellen (sowohl resorbierte und tote Föten als auch Nidationsstellen) berechnet. Für die Testsubstanz 18-Methyl-6α,7α-15β,16β-dimethylen-3-oxo-19-nor-17-pregn-4-en-21,17-carbolacton (Verb. 3a, siehe experimenteller Teil) wurde dabei eine schwangerschaftserhaltende Dosis (ED₅₀) von 120 µg/kg/Tag ermittelt. Für Drospirenon beträgt dieser Wert 3,5 mg/kg/Tag.

Die erfindungsgemäßen Verbindungen der allgemeinen Formel I verfügen über sehr starke gestagene Wirksamkeit bei gleichzeitiger schwacher Bindung an den Androgenrezeptor (Dissoziation).

Es wurde außerdem gefunden, daß erfindungsgemäße Verbindungen eine Kaliumretinierende, natriuretische (antimeralcorticoide) Wirkung in adrenalektomierten Ratten zeigen.

Aufgrund ihrer gestagenen Wirksamkeit können die neuen Verbindungen der allgemeinen Formel I allein oder in Kombination mit Estrogen in pharmazeutischen Präparaten zur Kontrazeption verwendet werden.
Wegen ihres günstigen Wirkungsprofils sind die erfindungsgemäßen Verbindungen besonders gut geeignet zur Behandlung prämenstrueller Beschwerden, wie Kopfschmerzen, depressive Verstimmungen, Wasserretention und Mastodynie.

Die Dosierung der erfindungsgemäßen Verbindungen in Kontrazeptionspräparaten soll 0,01 bis 5 mg, vorzugsweise 0,01 bis 2 mg pro Tag betragen.

Die Tagesdosis bei der Behandlung prämenstrueller Beschwerden liegt bei etwa 0.1 bis 20 mg.

Die gestagenen und estrogenen Wirkstoffkomponenten werden in Kontrazeptionspräparaten vorzugsweise zusammen oral appliziert. Die tägliche Dosis wird vorzugsweise einmalig verabfolgt.

Als Estrogene kommen synthetische Estrogene, vorzugsweise Ethinylestradiol, aber auch Mestranol in Betracht.

Das Estrogen wird in einer täglichen Menge verabfolgt, die der von 0.01 bis 0.04 mg Ethinylestradiol entspricht.

Die neuen Verbindungen der allgemeinen Formel I können auch in pharmazeutischen Präparaten zur Behandlung prä-, peri- und postmenopausaler Beschwerden sowie in Präparaten für die Hormon-Substitutionstherapie (HRT) eingesetzt werden.

Als Estrogene in derartigen Präparaten kommen in erster Linie natürliche Estrogene zur Anwendung, vor allem das Estradiol oder dessen Ester, beispielsweise Estradiolvalerat oder auch konjugierte Estrogene (CEEs = Conjugated Equine Estrogens), wie sie beispielsweise im Präparat PREMARIN^{®} enthalten sind.

Die Formulierung der pharmazeutischen Präparate auf Basis der neuen Verbindungen erfolgt in an sich bekannter Weise, indem man den Wirkstoff, gegebenenfalls in Kombination mit einem Estrogen, mit den in der Galenik gebräuchlichen Trägersubstanzen, Verdünnungsmitteln, gegebenenfalls Geschmackskorrigentien usw., verarbeitet und in die gewünschte Applikationsform überführt.

Für die bevorzugte orale Applikation kommen insbesondere Tabletten, Dragees, Kapseln, Pillen, Suspensionen oder Lösungen in Frage.

Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Es ist auch möglich, die erfindungsgemäßen Substanzen in ein transdermales System einzuarbeiten und sie damit transdermal zu applizieren.

Die neuen Verbindungen der allgemeinen Formel I werden erfindungsgemäß wie nachstehend beschrieben hergestellt. Die Syntheseroute für die neuartigen 18-Methyl-6,7-15,16-dimethylen-3-oxo-19-nor-17-pregn-4-en-21,17-carbolactone gemäß Schema 1 geht zum Beispiel von Verbindung 1 (DE 3402329) aus.
Die Einführung einer Δ⁶-Doppelbindung erfolgt zum Beispiel über Bromierung des 3,5-Dienamins oder durch eine modifizierte Dienoletherbromierung sowie anschließende Bromwasserstoffabspaltung (siehe z. B.J. Fried, J.A. Edwards, Organic Reactions in Steroid Chemistry, von Nostrand Reinhold Company 1972, S. 265-374).

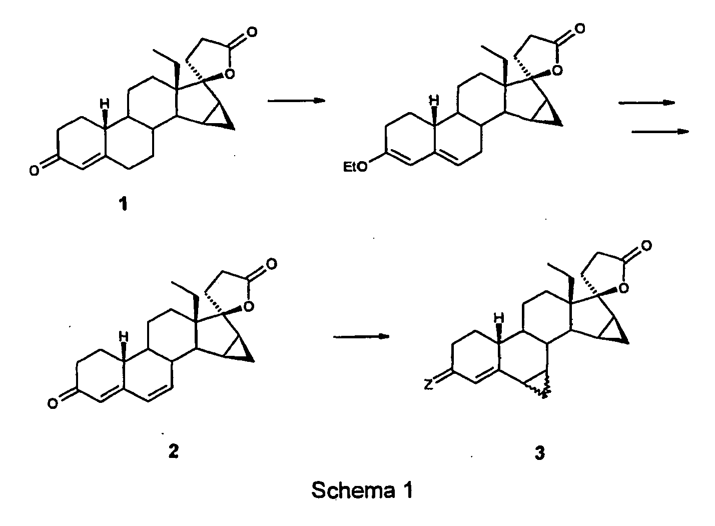

Die Dienoletherbromierung kann z. B. analog der Vorschrift von J.A. Zderic, Humberto Carpio, A. Bowers und Carl Djerassi in Steroids 1, 233 (1963**)** erfolgen. Die Bromwasserstoffabspaltung gelingt durch Erhitzen der 6-Bromverbindung mit basischen Reagenzien, wie z. B. LiBr oder Li₂CO₃ in aprotischen Lösungsmitteln wie Dimethylformamid bei Temperaturen von 50-120°C oder aber indem die 6-Bromverbindungen in einem Lösungsmittel wie Collidin oder Lutidin erhitzt werden zu Verbindung 2 (Beispiel 1).

Verbindung 2 wird dann durch Methylenierung der Δ⁶-Doppelbindung nach bekannten Verfahren z.B. mit Dimethylsulfoxoniummethylid (siehe z. B. DE-A 11 83 500, DE-A 29 22 500, EP-A 0 019 690, US-A 4,291, 029; E. J. Corey und M. Chaykovsky, J.Am.Chem.Soc. 84, 867 (1962)) in eine Verbindung 3 umgewandelt, wobei ein Gemisch der α- und β-Isomeren (Verbindungen 3a/3b) erhalten wird (das Verhältnis ist abhängig von den verwendeten Substraten und liegt bei ca. 1:1), das z. B. durch Chromatographie in die einzelnen Isomeren getrennt werden kann.

Die Einführung eines Substituenten R⁴ kann zum Beispiel ausgehend von einer Verbindung der Formel 3 durch Epoxidierung der Δ⁴-Doppelbindung mit Wasserstoffperoxid unter alkalischen Bedingungen und Umsetzung der entstandenen Epoxide in einem geeignetem Lösungsmittel mit Säuren der allgemeinen Formel H-R⁴ behandelt, wobei -R⁴ ein Halogenatom oder ein Pseudohalogen sein kann, oder mit katalytischen Mengen Mineralsäure umsetzt und gegebenenfalls die erhaltenen 4-Bromverbindungen der allgemeinen Formel I (wobei R⁴ = Brom) mit 2,2-Difluor-2-(fluorsulfonyl)essigsäuremethylester in Dimethylformamid in Gegenwart von Kupfer(I)iodid umsetzt.

Die Einführung einer 6-Methylengruppe kann zum Beispiel ausgehend von einem 3-Amino-3,5-dien-Derivat durch Umsetzung mit Formalin in alkoholischer Lösung unter Bildung einer 6a-Hydroxymethylgruppe und anschließender saurer Wasserabspaltung z.B. mit Salzsäure in Dioxan/Wasser erfolgen. Die Wasserabspaltung kann aber auch in der Weise erfolgen, dass zunächst die Hydroxygruppe gegen eine bessere Fluchtgruppe ausgetauscht und dann eliminiert wird. Als Fluchtgruppen sind z. B. das Mesylat, Tosylat oder Benzoat geeignet (siehe DE-A 34 02 3291, EP-A. 0 150 157, US-A 4,584,288; K. Nickisch et al., J. Med. Chem. 34, 2464 (1991)).

Eine weitere Möglichkeit zur Herstellung der 6-Methylenverbindungen besteht in der direkten Umsetzung der 4(5) ungesättigten 3-Ketone mit Acetalen des Formaldehyds in Gegenwart von Natriumacetat mit z. B. Phosphoroxychlorid oder Phosphorpentachlorid in geeigneten Lösungsmitteln wie Chloroform (siehe z. B. K. Annen, H. Hofmeister, H. Laurent und R. Wiechert, Synthesis 34 (1982)).

Die 6-Methylenverbindungen können zur Darstellung von Verbindungen der allgemeinen Formel 1, In denen R⁶ gleich Methyl ist und R⁶ und R⁷ gemeinsam eine zusätzliche Bindung bilden, genutzt werden.

Hierzu kann man z. B. ein von D. Burn et al. in Tetrahedron 21, 1619 (1965) beschriebenes Verfahren anwenden, bei dem eine Isomerisierung der Doppelbindung durch Erwärmen der 6-Methylenverbindungen in Ethanol mit 5% Palladium-Kohle-Katalysator, der entweder mit Wasserstoff oder durch Erwärmen mit einer geringen Menge Cyclohexen vorbehandelt wurde, erzielt werden. Die Isomerisierung kann auch mit einem nicht vorbehandelten Katalysator erfolgen, wenn zur Reaktionsmischung eine geringe Menge Cyclohexen zugesetzt wird. Das Auftreten geringer Anteile hydrierter Produkte kann durch Zugabe eines Überschusses an Natriumacetat verhindert werden.

Die Darstellung von 6-Methyl-4,6-dien-3-on-Derivaten kann aber auch direkt erfolgen (siehe K. Annen, H. Hofmeister, H. Laurent und R. Wiechert, *Lieb. Ann.* 712 (**1983**)).

Verbindungen, in denen R⁶ eine α-Methylfunktion darstellt, können aus den 6-Methylenverbindungen durch Hydrierung unter geeigneten Bedingungen dargestellt werden. Die besten Ergebnisse (selektive Hydrierung der exo-Methylenfunktion) werden durch Transfer-Hydrierung erreicht (E.A. Brande, R.P. Linstead und P.W.D. Mitchell, J. Chem.Soc. 3578 (1954)). Erhitzt man die 6-Methylenderivate in einem geeigneten Lösungsmittel, wie z. B. Ethanol, in Gegenwart eines Hydriddonators, wie z. B. Cyclohexen, so kommt man in sehr guten Ausbeuten zu 6α-Methylderivaten. Geringe Anteile an 6β-Methylverbindung können sauer isomerisiert werden (siehe z. B. D. Burn, D. N. Kirk und V. Petrow, Tetrahedron 1619(1965)).

Auch die gezielte Darstellung von 6β-Alkylverbindungen ist möglich. Hierfür werden die 4(5)-ungesättigten 3-Ketone z. B. mit Ethylenglycol, Trimethylorthoformiat in Dichlormethan in Gegenwart katalytischer Mengen einer Säure, (z. B. p-Toluolsulfonsäure) zu den entsprechenden 3-Ketalen umgesetzt. Während dieser Ketalisierung isomerisiert die Doppelbindung in die Position 5(6). Eine selektive Epoxidierung dieser 5(6)-Doppelbindung gelingt z. B. durch Verwendung organischer Persäuren, z. B. m-Chlorperbenzoesäure, in geeigneten Lösungsmittel wie Dichlormethan. Alternativ hierzu kann die Epoxidierung auch mit Wasserstoffperoxid in Gegenwart von z. B. Hexachloraceton oder 3-Nitrotrifluoracetophenon erfolgen. Die gebildeten 5,6α-Epoxide können dann unter Verwendung entsprechender Alkylmagnesiumhalogenide oder Alkyllithiumverbindungen axial geöffnet werden. Man gelangt so zu 5α-Hydroxy-6β-Alkylverbindungen. Die Spaltung der 3-Ketoschutzgruppe kann unter Erhalt der 5α-Hydroxyfunktion durch Behandeln unter milden sauren Bedingungen (Essigsäure oder 4 n Salzsäure bei 0°C) erfolgen. Basische Eliminierung der 5α-Hydroxyfunktion mit z. B. verd. wäßriger Natronlauge ergibt die 3-Keto-4-en-Verbindungen mit einer β-ständigen 6-Alkylgruppe. Alternativ hierzu ergibt die Ketalspaltung unter drastischeren Bedingungen (wäßrige Salzsäure oder eine andere starke Säure) die entsprechenden 6α-Alkylverbindungen.

Die erhaltenen Verbindungen der allgemeinen Formel I, in denen Z für ein Sauerstoffatom steht, können gewünschten falls durch Umsetzung mit Hydroxylaminhydrochlorid in Gegenwart eines tertiären Amins bei Temperaturen zwischen -20 und +40°C in ihre entprechenden Oxime überführt werden (allgemeine Formel I mit Z in der Bedeutung von =NOH, wobei die Hydroxygruppe syn- oder antiständig sein kann). Geeignete tertiäre Basen sind beispielsweise Trimethylamin, Triäthylamin, Pyridin, N,N-Dimethylaminopyridin, 1,5- Diazabicyclo[4.3.0]non-5-en (DBN) und 1,5- Diazabicyclo[5.4.0]undec-5-en (DBU), wobei Pyridin bevorzugt ist. Dies geht analog wie es in WO 98/24801 für die Herstellung entsprechender 3-Oxyimino-Derivate des Drospirenons beschrieben ist.

Die Entfernung der 3-Oxogruppe zur Herstellung eines Endprodukts der allgemeinen Formel I mit Z in der Bedeutung von zwei Wasserstoffatomen kann beispielsweise nach der in DE-A 28 05 490 angegebenen Vorschrift durch reduktive Spaltung eines Thioketals der 3-Ketoverbindung erfolgen.

Die nachfolgenden Beispiele dienen der näheren Erläuterung der Erfindung:

### Beispiel 1

### 18-Methyl-15β,16β-methylen-3-oxo-19-nor-17-pregna-4,6-dien-21,17-carbolacton

Zu einer Lösung von 11.0 g 18-Methyl-15β,16β-methylen-3-oxo-19-nor-17-pregn-4-en-21,17-carbolacton (DE3402329) in 110 ml Dioxan gab man 11 ml o-Ameisensäuretriethylester sowie 11 ml Dioxan / Schwefelsäure (12+0.42). Man erhielt 14.4 g 3-Ethoxy-18-methyl-15β,16β-methylen-19-nor-17-pregna-3,5-dien-21,17-carbolacton als Rohprodukt. Dieses wurde in 380 ml Aceton gelöst, mit 2.3 ml Pyridin, 10.3 g Natriumacetat, 28 ml Wasser sowie 7.6 g N-Bromsuccinimid versetzt, und 0.5 Stunden bei Eisbadtemperatur gerührt. Anschließend rührte man in Eiswasser ein, filtrierte den Niederschlag ab, wusch mit Wasser, nahm den Niederschlag in Dichlormethan auf, wusch mit Wasser, trocknete über Natriumsulfat und engte im Vakuum ein. Man erhielt 17 g 6-Brom-18-methyl-3-oxo-15β,16β-methylen-19-nor-17-pregn-4-en-21,17-carbolacton als Rohprodukt. Dieses wurde in 170 ml Dimethylformamid gelöst, mit 6.65 g Lithiumbromid sowie 7.87 g Lithiumcarbonat eine Stunde bei 100 °C gerührt. Anschließend rührte man in Eiswasser ein, filtrierte den Niederschlag ab, wusch mit Wasser, nahm den Niederschlag in Dichlormethan auf, wusch mit Wasser, trocknete über Natriumsulfat und engte im Vakuum ein. Nach Chromatographie an Keiselgel mit Hexan/Aceton erhält man 6.5 g 18-Methyl-15β,16β-methylen-3-oxo-19-nor-17-pregna-4,6-dien-21,17-carbolacton (Verbindung 2 in Schema 1) vom Schmelzpunkt 187 °C

### Beispiel 2

### 18-Methyl-6α,7α-15β,16β-dimethylen-3-oxo-19-nor-17-pregn-4-en-21,17-carbolacton

Eine Lösung von 5.8 g 18-Methyl-15β,16β-methylen-3-oxo-19-nor-17-pregna-4,6-dien-21,17-carbolacton in 200 ml Dimethylsulfoxid wurde mit 9.06 g Trimethylsulfoxoniumiodid und 1.613 g Natriumhydrid (55%ige Suspension in Öl) versetzt, und 20 Stunden unter Argon bei Raumtemperatur gerührt. Anschließend rührte man in Eiswasser ein, stellte schwach sauer, filtrierte den Niederschlag ab, nahm den Niederschlag in Dichlormethan auf, wusch mit Wasser, trocknete über Natriumsulfat, engte im Vakuum ein, und chromatographierte an Kieselgel mit Hexan / Aceton. Nach Umkristallisation der Fraktion III aus 2-Propanot/Aceton erhielt man 0.8 g 18-Methyl-6α,7α-15β,16β-dimethylen-3-oxo-19-nor-17-pregn-4-en-21,17-carbotacton (Verbindung 3a) als Kristalle vom Schmelzpunkt 262 °C. [α]_{D} = +89.7 ° (Methanol, c = 10.15 mg/ml)

### Beispiel 3

### 18-Methyl-6β,7β-15β,16β-dimethylen-3-oxo-19-nor-17-pregn-4-en-21,17-carbolacton

Nach der Methode des Beispiels 2 erhielt man nach der Chromatographie als Fraktion IV 0.9 g 18-Methyl-6β,7β-15β,16β-dimethylen-3-oxo-19-nor-17-pregn-4-en-21,17-carbolacton (Verbindung 3b) als Feststoff vom Schmelzpunkt 189-190 °C. [α]_{D} = -121.4° (Chloroform, c = 10.7 mg/ml) und [α]_{D} = +137.9 ° (Methanol, c = 10.63 mg/ml)

### Beispiel 4

### 7α,18-Dimethyl-15β,16β-methylen-3-oxo-19-nor-17-pregn-4-en-21,17-carbotacton

In eine Lösung von 0.4 g 18-Methyl-15β,16β-methylen-3-oxo-19-nor-17-pregna-4,6-dien-21,17-carbolacton in 7 ml Tetrahydrofuran gab man bei Raumtemperatur 12 mg Kupfer-I-chlorid und rührte 10 Minuten bevor man auf -15 °C abkühlte, mit 75 mg Aluminiumchlorid versetzte, 30 Minuten bei dieser Temperatur rührte, mit 0.8 ml Methylmagnesiumbromidlösung (3 M in Diethylether) tropfenweise versetzte, und eine Stunde bei -10 °C rührte. Zur Aufarbeitung wurde die Reaktionsmischung mit bei -10 °C mit 4n-Salzsäure versetzt, 1,5 Stunden bei Raumtemperatur gerührt, auf Wasser gegeben, dreimal mit Essigester extrahiert, über Natriumsulfat getrocknet, i.Vak. eingeengt, und an Kieselgel mit Hexan / Essigester chromatographiert. Nach Umkristallisation der Fraktion I erhielt man 182 mg 7α,18-Dimethyl-15β,16β-methylen-3-oxo-19-nor-17-pregn-4-en-21,17-carbolacton als Kristalle vom Schmelzpunkt 250 °C. [α]_{D}= 53.1 +/-0.3 ° (Chloroform, c = 10.3 mg/ml)

### Beispiel 5

### 7β,18-Dimethyl-15β,16β-methylen-3-oxo-19-nor-17-pregn-4-en-21,17-carbolacton

Nach der Methode des Beispiels 4 erhielt man nach der Chromatographie als Fraktion II 36 mg 7β,18-Dimethyl-15β,16β-methylen-3-oxo-19-nor-17-pregn-4-en-21,17-carbolacton als Feststoff vom Schmelzpunkt 226 °C. [α]_{D} = 9.0 +/-0.5° (Chloroform, c =10.2 mg/ml).

### Beispiel 6

### 7α-Ethyl-18-methyl-15β,16β-methylen-3-oxo-19-nor-17-pregn-4-en-21,17-carbolacton

In eine Lösung von 0.5 g 18-Methyl-15β,16β-methylen-3-oxo-19-nor-17-pregna-4,6-dien-21,17-carbolacton in 10 ml Tetrahydrofuran gab man bei Raumtemperatur 15 mg Kupfer-I-chlorid und rührte 10 Minuten bevor man auf-15 °C abkühlte, mit 93 mg Aluminiumchlorid versetzte, 30 Minuten bei dieser Temperatur rührte, mit 1.0 ml Ethylmagnesiumbromidlösung (3 M in Diethylether) tropfenweise versetzte, und eine Stunde bei -10°C rührte. Zur Aufarbeitung wurde die Reaktionsmischung mit bei -10 °C mit 3 ml 2n-Salzsäure versetzt, 0.5 Stunden bei Raumtemperatur gerührt, auf Wasser gegeben, dreimal mit Essigester extrahiert, über Natriumsulfat getrocknet, i.Vak. eingeengt, und an Kieselgel mit Hexan / Essigester chromatographiert. Nach Umkristallisation der Fraktion I erhielt man 180 mg 7α-Ethyl-18-methyl-15β,16β-methylen-3-oxo-19-nor-17-pregn-4-en-21,17-carbolacton
als Kristalle vom Schmelzpunkt 205-208 °C. [α]_{D} = +34.4 +/-0.3 ° (Chloroform, c = 10.3 mg/ml)

### Beispiel 7

### 7β-Ethyl-18-methyl-15β,16β-methylen-3-oxo-19-nor-17-pregn-4-en-21,17-carbolacton

Nach der Methode des Beispiels 6 erhielt man nach der Chromatographie als Fraktion II 110 mg 7β-Ethyl=18-methyl-15β,16β-methylen-3-oxo-19-nor-17-pregn-4-en-21,17-carbolacton als Feststoff vom Schmelzpunkt 169-171 °C. [α]_{D} = +30.8 +/-0.5° (Chloroform, c = 10.1 mg/ml).

### Beispiel 8

### 18-Methyl-15β,16β-methylen-3-oxo-7α-propyl-19-nor-17-pregn-4-en-21,17-carbolacton

In eine Lösung von 0.5 g 18-Methyl-15β,16β-methylen-3-oxo-19-nor-17-pregna-4,6-dien-21,17-carbolacton in 10 ml Tetrahydrofuran gab man bei Raumtemperatur 15 mg Kupfer-I-chlorid und rührte 10 Minuten bevor man auf -15°C abkühlte, mit 93 mg Aluminiumchlorid versetzte, 30 Minuten bei dieser Temperatur rührte, mit 1.5 ml Propylmagnesiumbromid-lösung (2 M in Tetrahydrofuran) tropfenweise versetzte, und eine Stunde bei -10 °C rührte. Zur Aufarbeitung wurde die Reaktionsmischung mit bei-10 °C mit 3 ml 2n-Salzsäure versetzt, 0.5 Stunden bei Raumtemperatur gerührt, auf Wasser gegeben, dreimal mit Essigester extrahiert, über Natriumsulfat getrocknet, i.Vak. eingeengt, und an Kieselgel mit Hexan / Essigester chromatographiert. Nach Umkristallisation der Fraktion I erhielt man 177 mg 18-Methyl-15β,16β-methylen-3-oxo-7α-propyl-19-nor-17-pregn-4-en-21,17-carbolacton
als Kristalle vom Schmelzpunkt 167.5 °C. [α]_{D} = +31.2 +/-0.3 ° (Chloroform, c = 10.1 mg/ml)

### Beispiel 9

### 18-Methyl-15β,16β-methylen-3-oxo-7β-propyl-19-nor-17-pregn-4-en-21,17-carbolacton

Nach der Methode des Beispiels 8 erhielt man nach der Chromatographie als Fraktion II 105 mg 18-Methyl-15β,16β-methylen-3-oxo-7β-propyl-19-nor-17-pregn-4-en-21.17-carbolacton als Feststoff vom Schmelzpunkt 90.9 °C. [α]_{D} = +27.4 +/-0.4° (Chloroform, c = 10.3 mg/ml).

## Patentansprüche

1. 18-Methyl-19-nor-17-pregn-4-en-21,17-carbolactone der allgemeinen Formel I 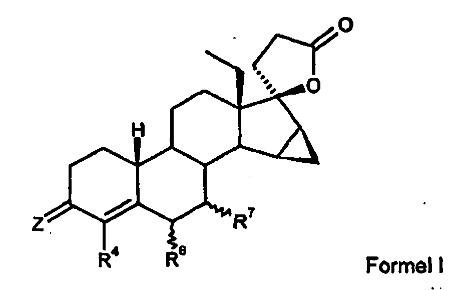 worin
Z ein Sauerstoffatom, zwei Wasserstoffatome, eine Gruppierung =NOR oder =NNHSO₂R, wobei R ein Wasserstoffatom oder eine gerad- oder verzweigtkettige Alkylgruppe mit 1 bis 4 bzw. 3 bis 4 Kohlenstoffatomen ist,
R⁴ ein Wasserstoffatom, ein Halogenatom, eine Methyl- oder eine Trifluormethylgruppe,
R⁶ und/oder R⁷ α- oder β-ständig sein kann und unabhängig voneinander eine gerad- oder verzweigtkettige Alkylgruppe mit 1 bis 4 bzw. 3 bis 4 Kohlenstoffatomen oder R⁶ ein Wasserstoffatom und R⁷ eine α- oder β-ständige, gerad- oder verzweigtkettige Alkylgruppe mit 1 bis 4 bzw. 3 bis 4 Kohlenstoffatomen oder
R⁶ und R⁷ gemeinsam eine α- oder β-ständige Methylengruppe oder eine zusätzliche Bindung
bedeuten.

2. Verbindungen der allgemeinen Formel I nach Anspruch 1, worin Z für ein Sauerstoffatom steht.

3. Verbindungen der allgemeinen Formel I nach Anspruch 1, worin Z für eine Gruppierung=NOR oder =NNHSO₂R und R darin für ein Wasserstoffatom steht.

4. Verbindungen der allgemeinen Formel I nach Anspruch 1, worin R⁴ ein Wasserstoffatom ist.

5. Verbindungen der allgemeinen Formel I nach Anspruch 1, worin R⁴ ein Chloratom ist.

6. Verbindungen der allgemeinen Formel I nach Anspruch 1, worin R⁶ für ein Wasserstoffatom und R⁷ für eine Methylgruppe stehen.

7. Verbindungen der allgemeinen Formel I nach Anspruch 1, worin R⁶ für ein Wasserstoffatom und R⁷ für eine Propylgruppe stehen.

8. Verbindungen der allgemeinen Formel I nach Anspruch 1, worin R⁶ und R⁷ gemeinsam für eine Methylengruppe stehen.

9. Verbindungen der allgemeinen Formel I nach Anspruch 1, worin R⁶ und R⁷ gemeinsam für eine Doppelbindung stehen.

10. Verbindungen der allgemeinen Formel I nach Anspruch 1, nämlich
18-Methyl-15β,16β-methylen-3-oxo-19-nor-17-pregna-4,6-dien-21,17-carbolacton,
18-Methyl-6α,7α-15β,16β-dimethylen-3-oxo-19-nor-17-pregn-4-en-21,17-carbolacton,
18-Methyl-6β,7β-15β,16β-dimethylen-3-oxo-19-nor-17-pregn-4-en-21,17-carbolacton,
7α,18-Dimethyl-15β,16β-methylen-3-oxo-19-nor-17-pregn-4-en-21,17-carbolacton,
7β,18-Dimethyl-15β,16β-methylen-3-oxo-19-nor-17-pregn-4-en-21,17-carbolacton,
3-Hydroxylamino-18-methyl-6β,7β-15β,16β-dimethylen-19-nor-17-pregn-4-en-21,17-carbolacton,
4-Chlor-18-methyl-6β,7β-15β,16β-dimethylen-3-oxo-19-nor-17-pregn-4-en-21,17-carbolacton
7α-Ethyl-18-methyl-15β,16β-methylen-3-oxo-19-nor-17-pregn-4-en-21,17-carbolacton
7β-Ethyl-18-methyl-15β,16β-methylen-3-oxo-19-nor-17-pregn-4-en-21,17-carbolacton
18-Methyl-15β,16β-methylen-3-oxo-7α-propyl-19-nor-17-pregn-4-en-21,17-carbolacton
18-Methyl-15β,16β-methylen-3-oxo-7β-propyl-19-nor-17-pregn-4-en-21,17-carbotacton.

11. Pharmazeutische Präparate enthaltend mindestens eine Verbindung gemäß Anspruch 1 sowie einen pharmazeutisch unbedenklichen Träger.

12. Pharmazeutische Präparate nach Anspruch 11, außerdem enthaltend mindestens ein Estrogen.

13. Pharmazeutische Präparate nach Anspruch 12 enthaltend Ethinylestradiol.

14. Pharmazeutische Präparate nach Anspruch 12, enthaltend ein natürliches Estrogen.

15. Pharmazeutische Präparate nach Anspruch 14, enthaltend Estradiol.

16. Pharmazeutische Präparate nach Anspruch 14, enthaltend Estradiolvalerat.

17. Pharmazeutische Präparate nach Anspruch 14, enthaltend mindestens ein konjugiertes Estrogen.

## Claims

1. 18-Methyl-19-nor-17-pregn-4-ene-21,17-carbolactones of general formula I 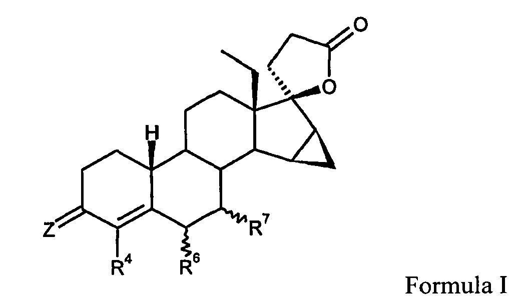 in which
Z means an oxygen atom, two hydrogen atoms, a grouping =NOR or =NNHSO₂R, whereby R is a hydrogen atom or a straight-chain or branched-chain alkyl group with 1 to 4 or 3 to 4 carbon atoms,
R⁴ is a hydrogen atom, a halogen atom, a methyl group, or a trifluoromethyl group,
R⁶ and/or R⁷ can be in α or β-position, and, independently of one another, mean a straight-chain or branched-chain alkyl group with 1 to 4 or 3 to 4 carbon atoms, or
R⁶ means a hydrogen atom and R⁷ means an α or β-position, straight- chain or branched-chain alkyl group with 1 to 4 or 3 to 4 carbon atoms, or
R⁶ and R⁷ together mean an α or β-position methylene group or an additional bond.

2. Compounds of general formula I according to claim 1, in which Z stands for an oxygen atom.

3. Compounds of general formula I according to claim 1, in which Z stands for a grouping =NOR or =NNHSO₂R, and R therein stands for a hydrogen atom.

4. Compounds of general formula I according to claim 1, in which R⁴ is a hydrogen atom.

5. Compounds of general formula I according to claim 1, in which R⁴ is a chlorine atom.

6. Compounds of general formula I according to claim 1, in which R⁶ stands for a hydrogen atom, and R⁷ stands for a methyl group.

7. Compounds of general formula I according to claim 1, in which R⁶ stands for a hydrogen atom and R⁷ stands for a propyl group.

8. Compounds of general formula I according to claim 1, in which R⁶ and R⁷ together stand for a methylene group.

9. Compounds of general formula I according to claim 1, in which R⁶ and R⁷ together stand for a double bond.

10. Compounds of general formula I according to claim 1, namely
18-Methyl-15β,16β-methylene-3-oxo-19-nor-17-pregna-4,6-diene-21,17-carbolactone
18-Methyl-6α,7α-15β,16β-dimethylene-3-oxo-19-nor-17-pregn-4-ene-21,17-carbolactone
18-Methyl-6β,7β-15β,16β-dimethylene-3-oxo-19-nor-17-pregn-4-ene-21,17-carbolactone
7a,18-Dimethyl-15β,16β-methylene-3-oxo-19-nor-17-pregn-4-ene-21,17-carbolactone
7β,18-Dimethyl-15β,16β-methylene-3-oxo-19-nor-17-pregn-4-ene-21,17-carbolactone
3-Hydroxylamino-18-methyl-6β,7β-15β,16β-dimethylene-19-nor-17-pregn-4-ene-21,17-carbolactone
4-Chloro-18-methyl-6β,7β-15β,16β-dimethylene-3-oxo-19-nor-17-pregn-4-ene-21,17-carbolactone
7α-Ethyl-18-methyl-15β,16β-methylene-3-oxo-19-nor-17-pregn-4-ene-21,17-carbolactone
7β-Ethyl-18-methyl-15β,16β-methylene-3-oxo-19-nor-17-pregn-4-ene-21,17-carbolactone
18-Methyl-15β,16β-methylene-3-oxo-7α-propyl-19-nor-17-pregn-4-ene-21,17-carbolactone
18-Methyl-15β,16β-methylene-3-oxo-7β-propyl-19-nor-17-pregn-4-ene-21,17-carbolactone.

11. Pharmaceutical preparations that contain at least one compound according to claim 1 as well as a pharmaceutically harmless vehicle.

12. Pharmaceutical preparations according to claim 11, in addition containing at least one estrogen.

13. Pharmaceutical preparations according to claim 12, containing ethinyl estradiol.

14. Pharmaceutical preparations according to claim 12, containing a natural estrogen.

15. Pharmaceutical preparations according to claim 14, containing estradiol.

16. Pharmaceutical preparations according to claim 14, containing estradiol valerate.

17. Pharmaceutical preparations according to claim 14, containing at least one conjugated estrogen.

## Revendications

1. 18-méthyl-19-nor-17-prégn-4-ène-21,17-carbolactones de formule générale I 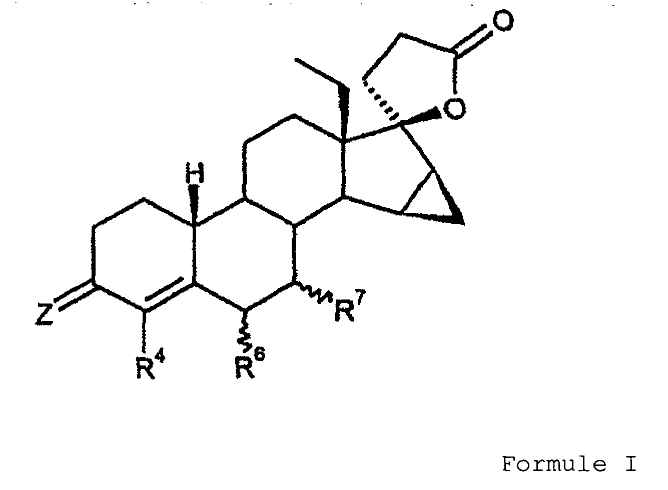 dans laquelle
Z représente un atome d'oxygène, deux atomes d'hydrogène, un groupement =NOR ou =NNHSO₂R, R étant un atome d'hydrogène ou un groupe alkyle à chaîne droite ou ramifiée ayant de 1 à 4 ou 3 à 4 atomes de carbone,
R⁴ représente un atome d'hydrogène, un atome d'halogène, un groupe méthyle ou trifluorométhyle,
R⁶ et/ou R⁷ peut/peuvent être en position α ou β et représentent, indépendamment l'un de l'autre, un groupe alkyle à chaîne droite ou ramifiée ayant de 1 à 4 ou 3 à 4 atomes de carbone, ou bien
R⁶ représente un atome d'hydrogène et R⁷ représente un groupe alkyle à chaîne droite ou ramifiée en position α ou β, ayant de 1 à 4 ou 3 à 4 atomes de carbone ou
R⁶ et R⁷ forment ensemble un groupe méthylène en position α ou β ou une liaison supplémentaire.

2. Composés de formule général I selon la revendication 1, dans lesquels Z représente un atome d'oxygène.

3. Composés de formule générale I selon la revendication 1, dans lesquels Z représente un groupement =NOR ou =NNHSO₂R et dans celui-ci R représente un atome d'hydrogène.

4. Composés de formule générale I selon la revendication 1, dans lesquels R⁴ est un atome d'hydrogène.

5. Composés de formule générale I selon la revendication 1, dans lesquels R⁴ est un atome de chlore.

6. Composés de formule générale I selon la revendication 1, dans lesquels R⁶ représente un atome d'hydrogène et R⁷ représente un groupe méthyle.

7. Composés de formule générale I selon la revendication 1, dans lesquels R⁶ représente un atome d'hydrogène et R⁷ représente un groupe propyle.

8. Composés de formule générale I selon la revendication 1, dans lesquels R⁶ et R⁷ représentent ensemble un groupe méthylène.

9. Composés de formule générale I selon la revendication 1, dans lesquels R⁶ et R⁷ représentent ensemble une double liaison.

10. Composés de formule générale I selon la revendication 1, à savoir
18-méthyl-15β,16β-méthylène-3-oxo-19-nor-17-prégna-4,6-diène-21,17-carbolactone,
18-méthyl-6α,7α-15β,16β-diméthylène-3-oxo-19-nor-17-prégn-4-ène-21,17-carbolactone,
18-méthyl-6β,7β-15β,16β-diméthylène-3-oxo-19-nor-17-prégn-4-ène-21,17-carbolactone,
7α,18-diméthyl-15β,16β-méthylène-3-oxo-19-nor-17-prégn-4-ène-21,17-carbolactone,
7β,18-diméthyl-15β,16β-méthylène-3-oxo-19-nor-17-prégn-4-ène-21,17-carbolactone,
3-hydroxylamino-18-méthyl-6β,7β-15β,16β-diméthylène-19-nor-17-prégn-4-ène-21,17-carbolactone,
4-chloro-18-méthyl-6β,7β-15β,16β-diméthylène-3-oxo-19-nor-17-prégn-4-ène-21,17-carbolactone,
7α-éthyl-18-méthyl-15β,16β-méthylène-3-oxo-19-nor-17-prégn-4-ène-21,17-carbolactone,
7β-éthyl-18-méthyl-15β,16β-méthylène-3-oxo-19-nor-17-prégn-4-ène-21,17-carbolactone,
18-méthyl-15β,16β-méthylène-3-oxo-7α-propyl-19-nor-17-prégn-4-ène-21,17-carbolactone,
18-méthyl-15β,16β-méthylène-3-oxo-7β-propyl-19-nor-17-prégn-4-ène-21,17-carbolactone.

11. Préparations pharmaceutiques contenant au moins un composé selon la revendication 1 ainsi qu'un véhicule pharmaceutiquement acceptable.

12. Préparations pharmaceutiques selon la revendication 11, en outre contenant au moins un estrogène.

13. Préparations pharmaceutiques selon la revendication 12, contenant de l'éthinylestradiol.

14. Préparations pharmaceutiques selon la revendication 12, contenant un oestrogène naturel.

15. Préparations pharmaceutiques selon la revendication 14, contenant de l'estradiol.

16. Préparations pharmaceutiques selon la revendication 14, contenant du valérate d'estradiol.

17. Préparations pharmaceutiques selon la revendication 14, contenant au moins un oestrogène conjugué.
